# EUROPEAN PATENT SPECIFICATION

(11) **EP 1 443 103 B1**
(45) Date of publication and mention of the grant of the patent: **09.12.2009**
(21) Application number: 03733199.8
(22) Date of filing: 30.05.2003
(51) Int. Cl.: C12M 3/00, C12M 1/00, C12N 5/00, C12N 5/06, A61L 27/38

(54) **CULTURING APPARATUS**
KULTIVIERUNGSVORRICHTUNG
DISPOSITIF DE CULTURE

(30) Priority: 13.06.2002 JP 2002172970
(43) Date of publication of application: 04.08.2004
(73) Proprietor: Olympus Corporation, Tokyo 151-0072 (JP)
(72) Inventor: HIBINO, Hiroki, Hachioji-shi, Tokyo 193-0811 (JP)
(74) Representative: von Hellfeld, Axel
(86) International application number: PCT/JP2003/006859
(87) International publication number: WO 2003/106613

(56) References cited:
- WO-A-97/40137
- JP-A- 3 103 258
- JP-A- 7 256 141
- JP-A- 2000 342 243
- JP-A- 2001 333 974
- US-A1- 2003 022 363

## Description

### Technical Field

The present invention relates to a culturing system for culturing cells from body fluid collected from a patient.

### Background Art

In recent years, it has become possible to repair areas of missing body tissue by regenerating bone or other body tissue by replenishing the site of missing body tissue such as a site of missing bone caused by the removal of bone tumors or trauma with a scaffold or other body tissue filler material. Although known examples of scaffolds include hydroxyapatite (HAP) and tricalcium phosphate (TCP), a scaffold composed of a porous calcium phosphate material like β-TCP is used based on the approach of not allowing foreign objects to remain in the body. If β-TCP is allows to contact osteocytes at a site of missing bone, osteoclasts consume the β-TCP enabling osteoblasts to form new bone. As a result, a process referred to as remodeling takes place. Namely, the scaffold used to replenish the site of missing bone is replaced by autologous bone over time.

On the other hand, in order to enhance the rate of repair of sites of missing bone following surgery, instead of using scaffold directly, bone marrow liquid collected from the patient is applied to the scaffold to replenish the site of missing bone. In this case, bone marrow liquid containing a large number of mesenchymal stem cells is collected from the patient, and after immersing the scaffold in this bone marrow liquid, it is applied to the site of missing bone. However, there is the problem of being unable to enable an adequate amount of mesenchymal stem cells to adhere to the scaffold simply by immersing the scaffold in the bone marrow liquid alone. Consequently, it has been considered to culture extracted bone marrow liquid and adhere the mesenchymal stem cells that grow to the scaffold so as to adhere an adequate amount of mesenchymal stem cells to the scaffold.

Various steps including a culturing step, media replacement step, infection examination step are included in the culturing of bone marrow liquid, and all of these steps have conventionally been performed manually.

However, manual intervention during these steps and during transfer between steps has the problem of increasing the risk of contamination by bacteria, fungi and other microorganisms.

Japanese published patent application no. 2001-333974 relates to an artificial material and a method of implanting same. However, improvements in the system for culturing the bone material are still sought.

### Disclosure of the Invention

In consideration of the aforementioned circumstances, the object of the present invention is to provide a culturing system capable of supplying healthy cultured cells by reducing opportunities for infection by bacteria and other microorganisms.

In order to achieve the aforementioned object, the present invention provides a culturing system as defined in claim 1. Preferred features of the invention are recited in the dependent claims.

In one embodiment, the culturing system of the invention comprises, in a sterile chamber, a storage unit that respectively stores media and body tissue filler, a cell extraction unit that extracts cells to be cultured from collected body fluid, a cell culturing unit that is connected to the storage unit and the cell extraction unit and cultures extracted cells in media to produce a body tissue filling composed of cultured cells and body tissue filler, and a sealing unit that seals the produced body tissue filling in a vessel.

According to this culturing system, collected body fluid is sent to a cell extraction unit, and cells to be cultured are extracted in said cell extraction unit. The extracted cells are sent to a cell culturing unit, and after being mixed with media stored in a storage unit, are grown by culturing for a predetermined amount of time. In addition, a body tissue filling is produced by adhering the grown cells to a body tissue filler stored in the storage unit. The produced body tissue filling is output sealed in a vessel by being sent to a sealing unit. In this case, since all of these steps are carried out in a sterile chamber, opportunities for infection by bacteria and other microorganisms are reduced through the entire culturing period, thereby making it possible to provide body tissue fillings in a healthy state.

In addition, the present invention provides the aforementioned culturing system that further comprises, in the sterile chamber, an examination unit that is connected to the cell extraction unit and the cell culturing unit, and at least examines body fluid and cells for infection.

According to this culturing system, examinations for infection can be carried out on body fluid and cells housed in the cell culturing unit. Since examinations for infection are also carried out within the sterile chamber, opportunities for infection by bacteria and other microorganisms in the inspection step are also reduced.

In addition, the present invention provides the aforementioned culturing system, wherein cells are examined for infection using media discarded from the cell culturing unit.

According to this culturing system, as a result of using media discarded from the cell culturing unit, cells can be examined for infection without contaminating the cultured cells. Since media is regularly or intermittently replaced in the cell culturing unit and media that is no longer necessary is discarded each time, by examining for infection using this discarded media, infection of cells that had previously contacted that media can be examined reliably.

In addition, the present invention provides the aforementioned culturing system, wherein the examinations for infection are carried out on a body tissue filling produced in the cell culturing unit.

According to this type of cell culturing unit, viable body tissue fillings can be provided more reliably by examining for inspection either by sampling the final product in the form of the body tissue filling by cutting away a portion of said filling, or by using a body tissue filling produced under the same conditions as the final product for examination.

In addition, the present invention provides the aforementioned culturing system further comprising a recording unit that is connected to the examination unit and records at least information that includes examination results on a vessel in which the body tissue filling is sealed in the sealing unit.

According to this type of culturing system, since information including the results of examinations for infection are recorded on the vessel in which the body tissue filling is sealed due to the operation of a recording unit, the person receiving the produced body tissue filling is able to determine the results of examination of said body tissue filling from information recorded on the vessel. Thus, the quality of body tissue fillings sealed in the vessel is guaranteed.

In addition, the present invention provides the aforementioned culturing system, wherein the cell culturing unit comprises a primary culturing unit that cultures extracted cells in media, and a secondary culturing unit that cultures the cultured cells in media together with a body tissue filler.

According to this type of culturing system, grown cultured cells can be obtained in the primary culturing unit, while a body tissue filling in which cultured cells have been grown using the body tissue filler as a base material can be obtained in the secondary culturing unit. Thus, cultured cells or body tissue fillings can be obtained as products. In addition, since examinations for infection can be carried out in both the primary and secondary culturing units, viability can be confirmed more reliably.

In addition, the present invention provides the aforementioned culturing system, wherein the sealing unit seals cells cultured in the primary culturing unit.

According to this type of culturing system, both body tissue fillings and cells cultured in the primary culturing unit can be provided in a viable state while avoiding infection by bacteria and other microorganisms.

### Brief Description of the Drawings

Fig. 1 is a schematic drawing showing and input and output of a culturing system as claimed in a first embodiment of the present invention.
Fig. 2 is a block diagram showing the general constitution of the culturing system of Fig. 1.
Fig. 3 is a flow chart for explaining each step of the culturing system of Fig. 2.
Figs. 4A and 4B are drawings for explaining the operation of a scaffold supply device used in the culturing system of Fig. 1.
Fig. 5 is a flow chart for explaining each step of a culturing system as claimed in a second embodiment of the present invention.
Fig. 6 is a schematic drawing showing an example of another culturing system of the present invention.

### Best Mode for Carrying Out the Invention

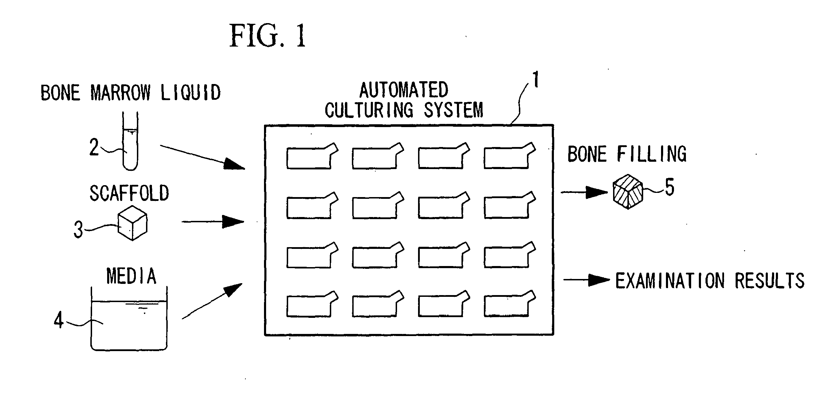

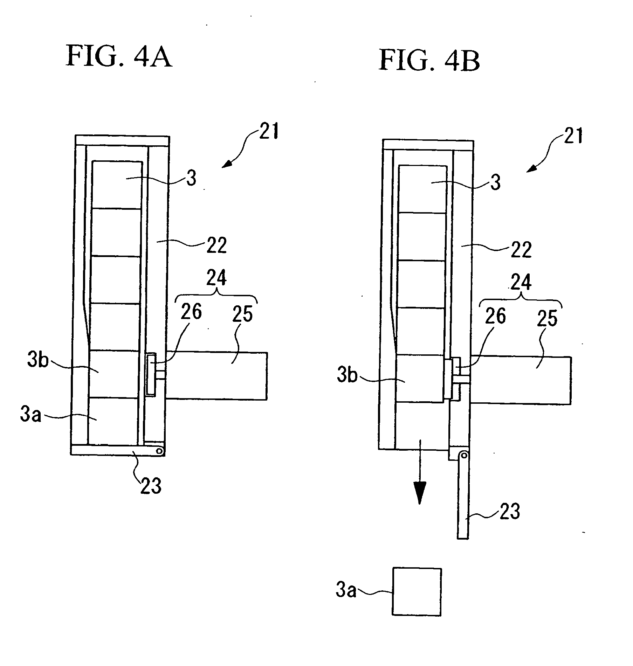

The following provides an explanation of a culturing system as claimed in a first embodiment of the present invention with reference to Figs. 1 through Figs. 4A and 4B.

As shown in Fig. 1, the culturing system as described in the present embodiment outputs a bone filling 5 to which cultured cells are adhered and data indicating the results of examinations for infection in each step by being provided with bone marrow liquid 2 collected from a patient, scaffold 3 to which cells contained in said bone marrow liquid 2 are adhered, and media 4.

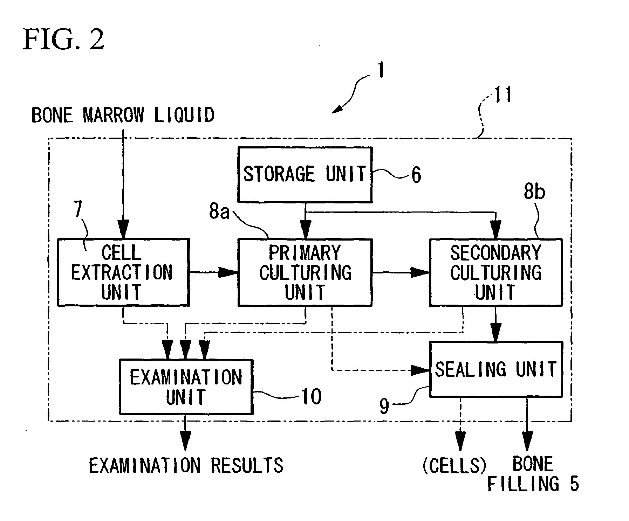

More specifically, as shown in Fig. 2, culturing system 1 as described in the present embodiment is equipped with a storage unit 6, a cell extraction unit 7, cell culturing units 8a and 8b, sealing unit 9 and examination unit 10 all within a sterile chamber 11.

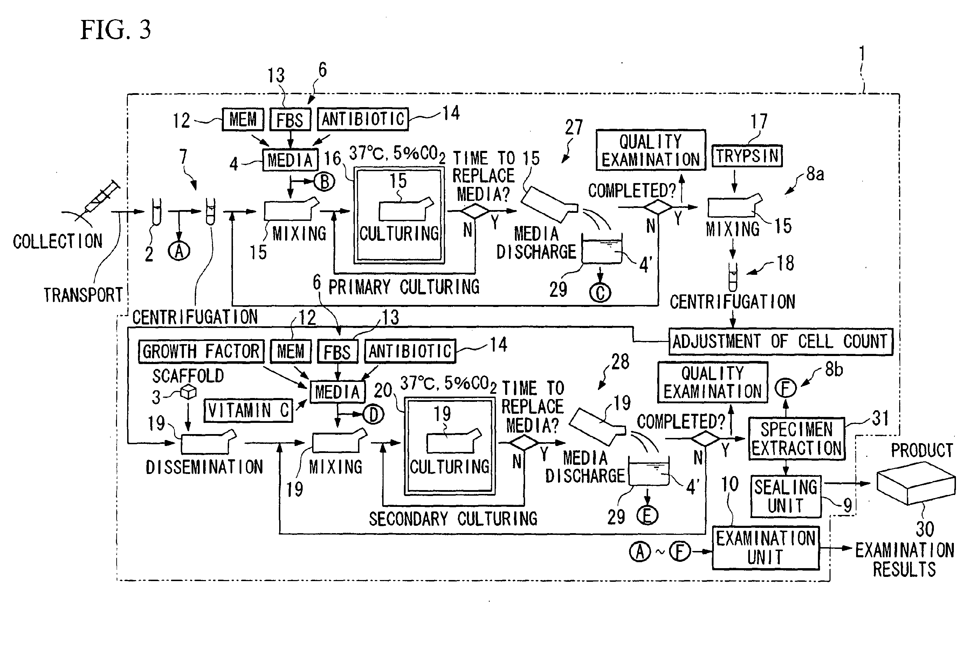

The aforementioned storage unit 6 is composed of a plurality of vessels that separately house scaffold 3 and media 4, respectively. As shown in Fig. 3, media 4 is composed of, for example, minimal essential medium (MEM), fetal bovine serum (FBS) and antibiotic, and these are housed either in a mixed state or in a state in which they are divided among separate vessels 12, 13 and 14. In addition, the vessel that stores media 4 is housed in a refrigeration device not shown at a temperature of, for example, 4°C. The antibiotic is, for example, a penicillin-based antibiotic.

Bone marrow liquid 2 is supplied to cell extraction unit 7. The aforementioned cell extraction unit 7 is, for example, a centrifuge, and is able to extract bone marrow cells having a large specific gravity from bone marrow liquid 2 by receiving bone marrow liquid 2 from a vessel in which bone marrow liquid 2 is housed followed by centrifuging that bone marrow liquid 2.

The cell culturing unit for culturing the aforementioned bone marrow cells is composed of primary culturing unit 8a and secondary culturing unit 8b. As shown in Fig. 3, primary culturing unit 8a is respectively connected to the aforementioned cell extraction unit 7 and the vessel housing media 4. This primary culturing unit 8a is equipped with a culturing vessel 15 for receiving bone marrow cells extracted in cell extraction unit 7 and media stored in a vessel, a stirring device (not shown) that stirs the bone marrow cells and media within culturing vessel 15, and a thermo-hygrostat 16 that maintains the stirred and mixed bone marrow liquid 2 and media 4 at a predetermined temperature (e.g., 37±0.5°C), CO₂ concentration (e.g., 5% by volume) and other culturing conditions, and is capable of culturing cells at fixed culturing conditions for predetermined amount of time. In addition, a vessel 17 that stores a protease such as trypsin is connected to primary culturing unit 8a. As a result, cells adhered to the bottom of culturing vessel 15 can be detached from culturing vessel 15 by supplying trypsin to culturing vessel 15 during the culturing step in primary culturing unit 8a. Moreover, a centrifuge 18 is also connected to primary culturing unit 8a, and this enables cells mixed in the trypsin to be extracted.

In addition to being connected to primary culturing unit 8a, secondary culturing unit 8b is also connected to a vessel (not shown) housing scaffold 3. Similar to the aforementioned primary culturing unit 8a, this secondary culturing unit 8b is equipped with a culturing vessel 19, stirring device not shown and a thermo-hygrostat 20. A scaffold supply device 21 like that shown in Figs. 4A and 4B, for example, is provided in the vessel that houses scaffold 3. This scaffold supply device 21 is composed of a shutter 23 provided in a vessel 22 that houses scaffold 3, and a holding device 24 that holds scaffold 3 farther up than a lowermost scaffold 3a so as to only permit dropping of this lowermost scaffold 3a. Holding device 24 is composed of a piston 25 and a holding rod 26.

Namely, holding device 24 is operated as shown in Fig. 4B from a state in which all scaffolds 3 are placed on shutter 23 as a result of closing said shutter 23 as shown in Fig. 4A, and by opening shutter 23 in the state in which the second scaffold 3b from the bottom is held by holding rod 26, only the lowermost scaffold 3a can be made to drop down. Thus, by operating holding device 24 and opening and closing shutter 23 in accordance with a requested amount of scaffolds 3 determined according to the amount of cultured bone marrow cells, a desired amount of scaffolds 3 are loaded into culturing vessel 19.

In addition, media replacement devices 27 and 28 for periodically replacing the media in media vessels 15 and 19 are provided in primary culturing unit 8a and secondary culturing unit 8b. Media replacement devices 27 and 28 are equipped with, for example, a vessel rotating device (not shown) that rotates culturing vessels 15 and 19 about the horizontal axis. Namely, media 4 inside media vessels 15 and 19 can be discharged from media vessels 15 and 19 by inclining or inverting media vessels 15 and 19 as a result of operating the vessel rotating device and rotating media vessels 15 and 19 about the horizontal axis.

In addition, a waste media vessel 29 that houses discharged media 4' is provided in media replacement devices 27 and 28.

Sealing unit 9 is connected to secondary culturing unit 8b, and is composed to output bone fillings produced in said secondary culturing unit 8b sealed within a sealed vessel 30.

Moreover, specimen culturing unit 31 is connected to secondary culturing unit 8b, and is composed so that specimens for examination are removed from the final products in the form of the bone fillings.

In addition, examination unit 10 is a fully automated, real-time polymerase chain reaction (PCR) device that is connected to the vessel of the aforementioned bone marrow liquid 2, waste media vessel 29 of primary culturing unit 8a, waste media vessel 29 of secondary culturing unit 8b and specimen extraction unit 31. In this fully-automated, real-time PCR device, primers are used so as to enable detection of all types of contaminants including fungi, bacteria, endotoxins, viruses and mycoplasma.

In the culturing system 1 as claimed in the present embodiment, the storage unit 6, cell extraction unit 7, cell culturing units 8a and 8b, sealing unit 9 and examination unit 10 are arranged within sterile chamber 11. Similar to a clean room, for example, the inside of sterile chamber 11 is set to a constant positive pressure, and the amount of dust per unit volume is limited by a particle counter and so forth.

The following provides an explanation of the operation of a culturing system 1 as claimed in the present embodiment composed in this manner.

According to culturing system 1 as claimed in the present embodiment, when bone marrow liquid 2 collected from a patient is placed in the bone marrow liquid vessel, a portion of said bone marrow liquid 2 is sent to examination unit 10 where it is examined for infection. The remainder of bone marrow liquid 2 is sent to cell extraction unit 7 where it is centrifuged in said cell extraction unit 7 and the bone marrow cells to be cultured are extracted.

The extracted bone marrow cells are grown to a predetermined number of cells by being sent to primary culturing unit 8a and cultured.

As shown in Fig. 3, in primary culturing unit 8a, the bone marrow cells that have been sent to this primary culturing unit 8a and media 4 composed of MEM, FBS, antibiotic and so forth that is stored in storage unit 6 are mixed within culturing vessel 15. A portion of media 4 is removed prior to mixing and then examined for infection by being sent to examination unit 10.

The mixed bone marrow cells and media 4 are cultured for a predetermined amount of time in a thermo-hygrostat in the state in which predetermined culturing conditions are maintained. A judgment is made as to the time when the media is to be replaced, and when a predetermined media replacement time is reached, media 4' is discharged to waste media vessel 29. A portion of media 4' that is discharged to waste media vessel 29 is examined for infection by being sent to examination unit 10. On the other hand, a judgment as to whether the time for completing primary culturing has been reached is made for the cells remaining in the culturing vessel, and if that completion time has not been reached, media 4 is mixed in again and primary culturing is continued. Mixing of media 4, culturing and discharge of media 4' are repeated until the time primary culturing is completed.

In the case the time for completion of primary culturing has been reached, a quality examination is carried out to determine whether or not the number of cells has reached the predetermined number, and in the case the number of cells has not increased sufficiently, the primary culturing step is carried out again. In the case the number of cells has been judged to have increased sufficiently in the quality examination, trypsin is charged into culturing vessel 15 to detach cultured cells that have grown on the bottom of culturing vessel 15. Subsequently, the number of cultured cells is adjusted by being applied to a hemocytometer, and then sent to secondary culturing unit 8b.

In secondary culturing unit 8b, together with the cultured cells that have been sent to this secondary culturing unit 8b being charged into culturing vessel 19, scaffold 3 housed in storage unit 6 is also charged into culturing vessel 19 resulting in dissemination of the cultured cells in scaffold 3. Next, similar to the case of primary culturing, media 4 is supplied to culturing vessel 19 from storage unit 6 and mixed therein. The supplied media 4 differs from the media 4 used in primary culturing in that, in addition to MEM, FBS and antibiotic, it is also mixed with differentiation inducing factor such as dexamethasone or β-glycerophosphate and nutrients such as vitamin C. Secondary culturing then begins in the state in which scaffold 3 disseminated with cultured cells in this manner is immersed in media 4. In this secondary culturing step as well, media 4' is discharged to waste media vessel 29 at predetermined media replacement intervals after which it is examined for infection. When the time for completion of secondary culturing is reached, secondary culturing is completed following another quality examination.

When secondary culturing is completed, a bone filling is output from secondary culturing unit 8b that has been adequately grown by using scaffold 3 as a base material. When the output bone filling is passed through specimen extraction unit 31, a portion of the bone filling is removed as a specimen for examination and then sent to examination unit 10 where it is examined for infection. In addition, the remainder of the bone filling is sent to sealing unit 9 where it is sealed within sealed vessel 30. The bone filling that has been output from sealing unit 9 and sealed in sealed vessel 30 is one of the final outputs of culturing system 1 as claimed in the present embodiment. In addition, the waste media 4' and specimens sent to examination unit 10 in each of the steps are examined for infection in examination unit 10, and those inspection results are also one of the final outputs of culturing system 1 as claimed in the present embodiment.

In this manner, according to the culturing system 1 as claimed in the present embodiment, a sealed bone filling and the results of infection examinations performed during culturing can be obtained simultaneously simply by charging bone marrow liquid 2 that has been collected from a patient. Thus, in comparison with the process that was performed manually in the prior art, the opportunities for infection by bacteria, fungi and other microorganisms can be reduced considerably. As a result, together with being able to provide viable bone fillings, the number of tasks that are performed by workers is reduced, thereby making it possible to easily produce a large quantity of bone fillings.

Furthermore, following culturing in the primary culturing unit, cells may also be sent to sealing unit 9 and output in a sealed state (route shown with the broken line in Fig. 2).

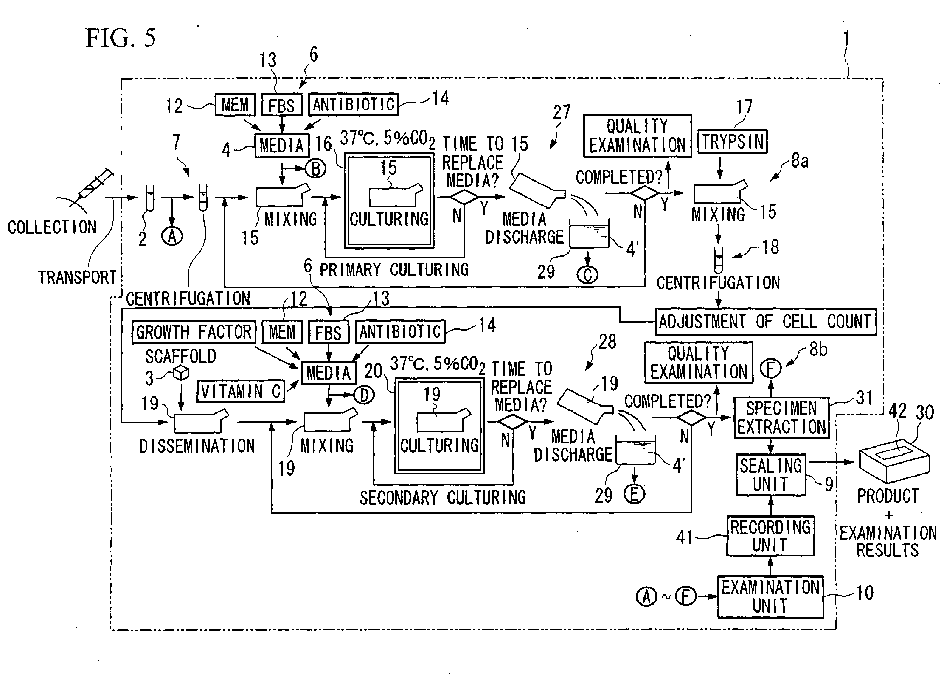

Next, an explanation is provided of a culturing system 40 as claimed in a second embodiment of the present invention with reference to Fig. 5.

Furthermore, in the explanation of the present embodiment, the same reference symbols are used for those locations in the second embodiment that share the same constitution as the culturing system 1 as claimed in the previously described first embodiment, and their explanation is omitted.

Culturing system 40 as claimed in the present embodiment differs from culturing system 1 as claimed in the first embodiment with respect to being provided with a recording unit 41 connected to examination unit 10. Recording unit 41 is provided with, for example, a database that correlates and stores groups of examination results for each bone filling and an identification code 42 for identifying those groups, and an identification code printer. An identification code 42 corresponding to a bone filling contained in sealed vessel 30 can be printed on said sealed vessel 30 in which said bone filling is sealed.

According to culturing system 40 as claimed in the present embodiment composed in this manner, a person who has received a sealed vessel 30 imprinted with an identification code 42 is able to extract the group of examination results corresponding to said identification code 42 simply by reading said identification code 42 imprinted on sealed vessel 30, thereby offering the effect of being able to easily determine the examination results at each stage of the culturing process for each bone filling in a form that corresponds with said bone filling.

Furthermore, the information stored in the database corresponding to identification code 42 may include not only the results of infection examinations for said bone filling, but also name, gender, age, date of birth and other identification data for identifying the patient, culturing conditions and culturing times that indicate a culturing log, and the results of examinations other than infection examinations, such as the results of quality examinations and so forth.

In addition, the transport devices between each of the units of culturing systems 1 and 40 arranged within sterile chamber 11, although not specifically indicated, should be composed by typical transport devices such as conveyors and manipulators. In addition, a roller pump and so forth can be employed for the device that supplies liquid media 4 and so forth.

In addition, as examples of components added to media 4, substances that contribute to growth such as growth factors such as cytokines, concentrated platelets, BMP, FGF, TGF-β, IGF, PDGF, VEGF, HGF and compounds thereof may be blended into media 4. In addition, hormone preparations such as estrogen and nutrients such as vitamins may also be blended into media 4.

In this case, the blending ratios of these growth factors, hormone preparations or nutrients should be determined corresponding to the degree of activity of the bone marrow liquid 2 to be cultured. In addition, although penicillin-based antibiotics are used for the antibiotic, cephams, macrolides, tetracyclines, fosfomycines, aminoglycosides, new quinolones and other arbitrary antibiotics may be used instead of these penicillin-based antibiotics.

In addition, although the explanation of the aforementioned embodiments using the example of bone marrow liquid 2 for the body fluid to be cultured, peripheral blood or placental blood may be used in its place. In addition, in this specification, liquids that contains somatic cells such as ES cells, somatic stem cells, mesenchymal cells, bone cells and chondrocytes are also referred to as body fluids for the sake of convenience. Moreover, the cells may be autologous cells or heterologous cells.

In addition, although bone was used as an example of body tissue in the aforementioned embodiments, fillings of other body tissues may also be produced. In this case, any arbitrary material having affinity with body tissue can be used for the body tissue filler, and a material that is absorbed by the body is even more preferable. In addition, this material may also be porous. Porous materials refer to porous ceramics, collagen, polylactic acid or porous metals and so forth having biocompatibility, and there are no restrictions on these materials provided they have a large number of pores. Examples of porous materials that can typically be used include calcium phosphate-based ceramics such as apatite and β-tricalcium phosphate (β-TCP), collagen or polylactic acid and so. In addition, calcium-phosphate-based ceramics may be combined with collagen, or calcium phosphate-based ceramics may be combined with polylactic acid. β-TCP, collagen and polylactic acid have the characteristic of being biodegradable and absorbed by the body, while apatite has the characteristic of having superior strength. It goes without saying that a person with ordinary skill in the art would be able to suitably select and use an appropriate type of porous material corresponding to the transplant site and so forth.

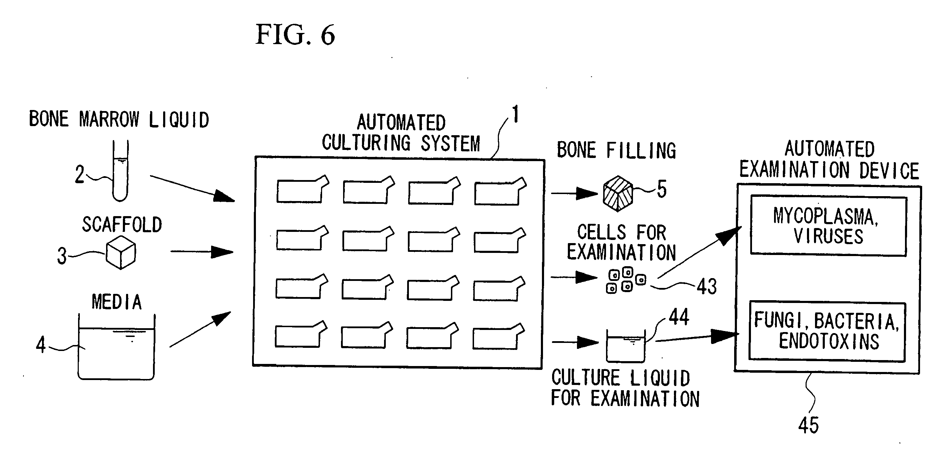

In addition, although human intervention has been completely eliminated from the culturing process in each of the aforementioned embodiments, as an example of an alternative to this, as shown in Fig. 6, bone filling 5, cells for examination 43 and media for examination 44 may be output from culturing system 1, while examinations for infection by fungi, bacteria and other microorganisms may be performed by an examination device 45 provided outside culturing system 1.

In addition, the concrete structures of each of the constituent devices such as scaffold supply device 21 in each of the aforementioned embodiments represent examples of one manner of composing the present invention, and the present invention is not limited to these.

### Industrial Applicability

As has been explained above, according to the culturing system as claimed in the present invention, since body tissue fillings can be produced simply by charging a body fluid without requiring human intervention in a sterile chamber, the effect is obtained of being able to provide viable body tissue fillings while reducing opportunities for infection.

In addition, since the examination results can be obtained along with the body tissue fillings, the effect is obtained of being able to enhance the reliability with respect to quality of the supplied body tissue fillings, thereby allowing a user to use the fillings with greater peace of mind.

## Claims

1. A culturing system (1) for use in a sterile chamber, comprising:
a storage unit (6) that respectively stores media (4) and body tissue filler(3),
a cell extraction unit (7) that extracts cells to be cultured from collected body fluid,
a cell culturing unit (8a, 8b) that is connected to the storage unit (6) and the cell extraction unit (7), and cultures extracted cells in media (4) to produce a body tissue filling (5) composed of cultured cells and body tissue filler (3), and
a body tissue filler supply device (21) for housing and supplying the body tissue filler (3), the body tissue filler supply device (21) comprising a shutter (23) for opening and closing the body tissue filler supply device (21) and comprising a holding device (24) for holding at least a part of the body tissue filler (3), wherein by operating the holding device (24) and the shutter (23) a desired amount of the body tissue filler (3) can be loaded into the cell culturing unit (8a, 8b).

2. A culturing system (1) according to claim 1, further comprising, in a sterile chamber, a sealing unit (9) that seals the produced body tissue filling (5) in a vessel.

3. A culturing system (1) according to claim 1 or claim 2, further comprising, in the sterile chamber (11), an examination unit (10) that is connected to the cell extraction unit (7) and the cell culturing unit (8a, 8b), and at least examines body fluid and cells for infection.

4. A culturing system (1) according to claim 3, wherein cells are examined for infection using media (4) discarded from the cell culturing unit (8a, 8b).

5. A culturing system (1) according to claim 3, wherein the examinations for infection are also carried out on a body tissue filling (5) produced in the cell culturing unit (8a, 8b).

6. A culturing system (1) according to claim 3, further comprising a recording unit (41) that is connected to the examination unit (10) and records at least information that includes examination results on a vessel in which the body tissue filling (5) is sealed in the sealing unit (9).

7. A culturing system (1) according to claim 2, wherein the cell culturing unit (8a, 8b) comprises a primary culturing unit (8a) that cultures extracted cells in media (4), and a secondary culturing unit (8b) that cultures the cultured cells in media (4) together with a body tissue filler (3).

8. A culturing system (1) according to claim 7, wherein the sealing unit (9) seals cells cultured in the primary culturing unit (8a).

## Patentansprüche

1. Kultivierungsvorrichtung (1) zur Verwendung in einer sterilen Kammer, umfassend:
eine Speichereinheit (6), die jeweils ein Medium (4) und einen Körpergewebefüller (3) speichert,
eine Zellextraktionseinheit (7), die von gesammelter Körperflüssigkeit zu kultivierende Zellen extrahiert,
eine Zellkultivierungseinheit (8a, 8b), die mit der Speichereinheit (6) und der Zellextraktionseinheit (7) verbunden ist und extrahierte Zellen in dem Medium (4) kultiviert, um eine aus den kultivierten Zellen und dem Körpergewebefüller (3) bestehende Körpergewebefüllung (5) herzustellen, und
eine Körpergewebefüllerzuführvorrichtung (21) zum Aufnehmen und Zuführen des Körpergewebefüllers (3), wobei die Körpergewebefüllerzuführvorrichtung (21) eine Verschlussklappe (23) zum Öffnen und Schließen der Körpergewebefüllerzuführvorrichtung (21) und eine Haltevorrichtung (24) zum Halten wenigstens eines Teils des Körpergewebefüllers (3) umfasst,
wobei durch Betätigung der Haltevorrichtung (24) und der Verschlussklappe (23) eine gewünschte Menge des Körpergewebefüllers (3) in die Zellkultivierungseinheit (8a, 8b) eingebracht werden kann.

2. Kultivierungsvorrichtung (1) nach Anspruch 1, weiterhin umfassend:
eine in einer sterilen Kammer vorgesehene Versiegelungseinheit (9), die die hergestellte Körpergewebefüllung (5) in einem Behälter versiegelt.

3. Kultivierungsvorrichtung (1) nach Anspruch 1 oder 2, weiterhin umfassend:
eine in der sterilen Kammer (11) vorgesehene Untersuchungseinheit (10), die mit der Zellextraktionseinheit (7) und der Zellkultivierungseinheit (8a, 8b) verbunden ist und wenigstens die Körperflüssigkeit und die Zellen auf eine Infektion hin untersucht.

4. Kultivierungsvorrichtung (1) nach Anspruch 3, wobei die Zellen auf eine Infektion hin untersucht werden unter Verwendung eines aus der Zellkultivierungseinheit (8a, 8b) ausgetragenen Mediums (4).

5. Kultivierungsvorrichtung (1) nach Anspruch 3, wobei die Untersuchungen auf eine Infektion hin auch bei einer in der Zellkultivierungseinheit (8a, 8b) hergestellten Körpergewebefüllung (5) ausgeführt werden.

6. Kultivierungsvorrichtung (1) nach Anspruch 3, weiterhin umfassend:
eine Erfassungseinrichtung (41), die mit der Untersuchungseinheit (10) verbunden ist und zumindest Informationen erfasst, die Untersuchungsergebnisse über einen Behälter, in dem die Körpergewebefüllung (5) in der Versiegelungseinheit (9) versiegelt ist, enthalten.

7. Kultivierungsvorrichtung (1) nach Anspruch 2, wobei die Zellkultivierungseinheit (8a, 8b) eine Primärkultivierungseinheit (8a), die extrahierte Zellen in dem Medium (4) kultiviert, und eine Sekundärkultivierungseinheit (8b), die die kultivierten Zellen in dem Medium (4) zusammen mit einem Körpergewebefüller (3) kultiviert, umfasst.

8. Kultivierungsvorrichtung (1) nach Anspruch 1, wobei die Versiegelungseinheit (9) in der Primärkultivierungseinheit (8a) kultivierte Zellen versiegelt.

## Revendications

1. Système de culture (1) pour une utilisation dans une chambre stérile, comprenant :
une unité de stockage (6) qui stocke respectivement un milieu (4) et une charge de tissu corporel (3),
une unité d'extraction cellulaire (7) qui extrait les cellules à cultiver du fluide corporel recueilli,
une unité de culture cellulaire (8a, 8b) qui est reliée à l'unité de stockage (6) et l'unité d'extraction cellulaire (7), et des cultures de cellules extraites dans du milieu (4) pour produire un matériau de remplissage de tissu corporel (5) composé de cellules cultivées et de charge de tissu corporel (3), et
un dispositif d'alimentation en charge de tissu corporel (21) pour recevoir et alimenter la charge de tissu corporel (3), le dispositif d'alimentation en charge de tissu corporel (21) comprenant un volet (23) pour ouvrir et fermer le dispositif d'alimentation en charge de tissu corporel (21) et comprenant un dispositif de maintien (24) pour contenir au moins une partie de la charge de tissu corporel (3), dans lequel en faisant fonctionner le dispositif de maintien (24) et le volet (23) une quantité souhaitée de la charge de tissu corporel (3) peut être chargée dans l'unité de culture cellulaire (8a, 8b).

2. Système de culture (1) selon la revendication 1, comprenant en outre, dans une chambre stérile, une unité de scellage (9) qui scelle le matériau de remplissage de tissu corporel produit (5) dans un récipient.

3. Système de culture (1) selon la revendication 1 ou la revendication 2, comprenant en outre, dans la chambre stérile (11), une unité d'analyse (10) qui est reliée à l'unité d'extraction cellulaire (7) et l'unité de culture cellulaire (8a, 8b), et analyse au moins le fluide corporel et les cellules à la recherche d'une infection.

4. Système de culture (1) selon la revendication 3, dans lequel des cellules sont analysées à la recherche d'une infection en utilisant le milieu (4) rejeté de l'unité de culture cellulaire (8a, 8b).

5. Système de culture (1) selon la revendication 3, dans lequel les analyses à la recherche d'une infection sont également réalisées sur un matériau de remplissage de tissu corporel (5) produit dans l'unité de culture cellulaire (8a, 8b).

6. Système de culture (1) selon la revendication 3, comprenant en outre une unité d'enregistrement (41) qui est reliée à l'unité d'analyse (10) et enregistre au moins une information qui inclut des résultats d'analyse sur un récipient dans lequel le matériau de remplissage de tissu corporel (5) est scellé dans l'unité de scellage (9).

7. Système de culture (1) selon la revendication 2, dans lequel l'unité de culture cellulaire (8a, 8b) comprend une unité de culture primaire (8a) qui cultive des cellules extraites dans du milieu (4), et une unité de culture secondaire (8b) qui cultive les cellules cultivées dans du milieu (4) conjointement à une charge de tissu corporel (3).

8. Système de culture (1) selon la revendication 7, dans lequel l'unité de scellage (9) scelle les cellules cultivées dans l'unité de culture primaire (8a).
